# EUROPEAN PATENT APPLICATION

(11) **EP 1 752 155 A1**
(43) Date of publication of application: **14.02.2007**
(21) Application number: 05735487.0
(22) Date of filing: 04.04.2005
(51) Int. Cl.: A61K 36/18, A61K 35/12, A61K 31/56, A61P 5/00

(54) **COMPOSITIONS FOR CORRECTING AGE RELATED CHANGES OF A HUMAN ENDOCRINE SYSTEM AND METHODS FOR PRODUCING A PHARMACEUTICAL FORM BASES ON SAID COMPOSITIONS**

(30) Priority: 26.04.2004 RU 2004112473
(71) Applicant: Obschestvo S Ogranichennoi Otvetstvennostyu "Rusgen", Moscow 123056 (RU)
(72) Inventor: MUSAEVA, Adilya Rafik kyzy, Moscow, 117279 (RU); SMIRNOVA, Olga Vyacheslavovna, Moscow, 111020 (RU); KUZINA, Irina Nikolaevna, Moskovskaya obl., 140700 (RU); BOGORAD, Roman Lvovich, Moscow, 117292 (RU); PERMINOVA, Irina Olegovna, Moscow, 109382 (RU)
(74) Representative: Stein-Dräger, Christiane
(86) International application number: PCT/RU2005/000164
(87) International publication number: WO 2005/102372

(57) **Abstract**

The present invention relates to anti-aging pharmacology and is intended for correction of age-related changes in human endocrine system. The essence of the invention is the compositions for correction of certain age-related endocrine changes and method for production of pharmaceutical form based thereon. Each composition contains a mixture of substances exhibiting hormonal activity (HA) or hormone-like activity (HLA) selected from the group: C21 or C 19 steroids, pregnenolone, 17-hydroxypregnenolone, DHEA, progesterone, testosterone, or a hormone-like substance of herbal origin (HS), and also a dry methanolic of Nettle root extract, taken in a definite ratio, wherein HA, HLA or HS with the Nettle root extract can further contain a Deer Antler velvet extract and/or Ginseng root extract, and HS can comprise diosgenin or protodioscin. One variant of the composition is a mixture of a dry Ginseng root extract and a dry Nettle root extract. The inventive method for production of said pharmaceutical form consists in mixing an active component and pharmaceutically acceptable carrier with suitable adjuvants in an effective amount. The useful result is in broadening the range of natural agents able to correct age-related hormonal changes in human organism.

## Description

### Field of the Art

The present invention relates to pharmacology of rejuvenescence and more particularly, development of compositions for correction of age-related changes in human endocrine system and method of production pharmaceutical forms based on such compositions.

Age-related changes arise along all lines of the vital activity of cells and tissues, and all of them lead to aging of the organism. Aging is a feature inherent to all multicellular organisms. It is characterized by disturbances in the functional capacities of an organism. This becomes particularly prominent at the end of a reproductive period which gradually passes into a period of aging. The latter period has an important distinctive feature: during this period reproduction cannot take place. Besides, the activity of all organs becomes reduced. A number of changes occurring on the molecular and cellular levels lead to disorders in the functioning of organs and of organism as a whole. The duration of the aging period cannot be determined precisely because it is not known at which moment of time disturbances of separate functions of an organism commence. If we adopt the disappearance of reproductive ability as the criterion of aging, it may be assumed that in females aging begins at age of about 45. It is known, however, that disturbances of some functions, e.g., muscular activity and respiration, occur both in men and women already at age of about 30 years. The time of onset, duration and speed of aging depend all on the reproductive period, and while the specific features of the latter are determined by the period of development. All the periods are interrelated. Consequently, aging cannot be considered as an isolated and independent period of life. Information related to the periods of development and reproduction is of great importance for understanding the processes of aging (M.S. Kanungo, "Biochemistry of Ageing", London: New York: Academic Press, 1980).

### Description of the Prior Art

At present a large number of medicinal agents exist that directed both against external manifestations of organism aging, on slowing down and even elimination of certain internal factors of aging. The point in hand will be mainly about substances of natural origin. Particularly, for elimination of external manifestations of skin senescence, it is proposed to use an extract from plants of the Iridaceae family as an active agent producing an activating effect on chlorine channels in order to loose or relax the tension of skin and/or subcutaneous tissues. The agent is more effective for reducing normal and small wrinkles (RU 2174010, 2001). Also similar agents are described in RU 2142784, 1999; RU 2202337, 2003; etc.

To achieve an overall health-improving effect on an organism, biologically active formulations and food supplements are used with adaptogenic and stimulating effect on the immune and antioxidant systems. For instance, a formulation of such kind is a complex containing a powder of Magnolia Vine fruits, a lyophilized royal jelly, a dry extract of propolis, Ppotassium iodide, ferrous gluconate, maltodextrin, dehydrated colloidal silica, and magnesium stearate (RU 2195952, 2003).

A biologically active supplement in the form of a balsam is known, which is an aqueous-alcoholic extract of a composition containing 20-25 wt.% in-shell pignoli nuts, 20-25 wt.% natural honey, and an aqueous-alcoholic liquid, wherein the end product is characterized by pH 4.6-5.2, 23-26 wt.% concentration of sugars, 15-35 vol.% concentration of ethyl alcohol, and 20-30 g/100 CM³ dry matter. The ready-to-use balsam contains ions of iron, manganese, copper, zinc, silver, cobalt, chromium, magnesium, calcium, sodium, potassium, molybdenum, and selenium (RU 21992348, 20003).

An agent has been developed for the prophylaxis of liver diseases and improvement of the overall physical and psychological condition in elderly humans. The agent comprises 60.0-95.0 wt.% of flower pollen, 5.0-40.0 wt.% of DNA (RU 2083218, 1997).

Another biologically active supplement contains a dry extract of purple Echinacea and physiologically acceptable additives: magnesium stearate, a finely dispersed powder of dry Echinacea herb, ascorbic acid, and microcrystalline cellulose in the following wt.% ratio of the ingredients: the dry extract of Echinacea herb and/or roots, 10.0-40.0; the powder of dry Echinacea herb, 20.0-70.0; ascorbic acid, 1.0-4.0 ; magnesium stearate, 0.3-1.0; microcrystalline cellulose, to 100% (RU 2182011, 2002). A composition is known, which comprises hematogen, "Decamevit", and also hormones: melatonin, dehydroepiandrosterone, testosterone (RU 2152744, 2000).

A medicinal agent is also known, which produces an immunocorrective effect, based on a thick plant extract produced from purple Echinacea, Beggartricks, and hill-growing Saltwort herbs, Nettle leaves, Licorice roots and Laminaria thallus, taken in a following wt.pts ratio: purple Echinacea herb, 50; Beggartricks herb, 10; hill-growing Saltwort herb, 10; Nettle leaves, 10; Licorice roots, 10; Laminaria thallus, 10. Besides, the medicinal agent can be produced in the form of granules containing 90% of lactose and 10% of a thick extract of the above-cited plants (RU 2203676, 2003).

"MILONA", a medicinal and health maintenance supplement, contains a main complex of plants, consisting of the roots and rhizomes of Licorice, Sweet flag and Elecampane, taken in a 2:1:1 ratio and produces an overall maintenance effect on an organism, as well as additional complexes of medicinal plants, taken in definite quantitative ratio to enhance separate effects and reactions of an organism. The supplement improves the function of the respiratory system; cardiovascular system; produces a sedative effect; decreases nervous excitation, irritability, insomnia, climacteric disorders, reduces hypertension, regulates and normalizes the activity of the liver and the gall bladder; regulates and normalize the activity of the urinary tracts; provides antisclerotic, cardiotonic, vasodilatory effect; stimulates the work the brain function; improves the memory; facilitates the concentration; vitalizes the sexual sphere in males and females; provides preventive and overall maintenance effect (RU 2178660, 2002).

However, mentioned agents have, mainly, a health maintenance effect and are not always directed on correction of age-related changes, their action is often associated with side effects in the form of arterial pressure dysregulation and allergic reactions. Polycomponent systems contain ingredients with different effects on an organism, and the efficiency of their concurrent use is questionable. Complex preparations containing substances of both vegetable and animal origin are much more effective. For example, the preparation "ALEVALON" contains (in weight percent): Elfwort roots, 2-5; Marsh Cinquefoil roots, 1-4; Birch buds, 0.5-1.5; ethyl alcohol, and the solvent. Alcoholic extract (10-15%) of Pantocrine or Ginseng can also be added to said formulation.(RU 2102998, 1998).

Some preparations used as an anti-aging for different categories of people suffering from definite diseases, and they cannot be used by all patients for correction of age-related changes in the endocrine system. For example, an agent for treatment of male sexual dysfunction (RU 21673805, 2001) or for the treatment of oncologic diseases, (RU Application No. 95122372, 1997; RU Application No. 200011296, 2003).

We believe that for our invention the most relevant analogs are those intended for use in patients for age-related corrections in the endocrine system and based on herbal preparations. Such are, e.g., French Patent No. 2671488, 1992: a composition containing a Ginseng extract and magnesium salts; RU 2169675, 2001: a preparation for prophylaxis of aging, containing long Turmeric, Wild Fennel, Myrobalan, common Ginger, Garlic, Cardamom, Cubeb Pepper, Long Pepper, Cinnamon, and sugar. (RU 2169575, 2001).

It has been suggested to use an extract of white Bryony (Bryonia alba L.) roots as an agent for prolongation of the life span in laboratory animals that maybe used in experimental biology and medicine for studying the mechanisms of aging and prolongation of the life span (RU2087153, 1997).

A biologically active supplement is also known, which contains aqueous-alcoholic extracts of Calendula flowers, Dill seeds, Black Currant leaves, Sage leaves, Noble Laurel leaves, Estragon herb, Hyssop herb, mouse-ear Hawkweed herb, Lycopodium spores, Blood-red Geranium root at a definite ratio of the components (RU 2176895, 2001). The supplement may also contain menthol alcohol and Rose oil. The method of enhancing the mental and physical working capacity of humans contemplates the use of said biologically active supplement for enhancing the organism resistance to unfavorable environmental factors. The invention is recommended for the prophylaxis of cardiovascular, infectious and oncological diseases as well as prevention of premature aging.

One of the most up-to-date analogs based on herbal mixtures is a herbal agent enabling to prevent of aging processes and comprising detoxifying species and rejuvenative species, wherein the detoxifying species comprise tomentose Burdock roots, Sickle Alfalfa herb and Meadow Clover flowers taken in the following weight percent ratio of the components: Tomentose burdock roots, 10.0; Sickle Alfalfa herb, 35.0-40.0; Meadow Clover flowers, 50.0-55.0. The rejuvenative mixture contains Garlic bulbs, and fruits of Lemon, Honeysuckle, Blueberry, Buckleberry and Cowberry offic. taken in the following weight percent ratio of the components: Garlic bulbs, 20.0-24.0; Lemon fruits, 20.0-24.0; Honeysuckle fruits, 13.0-15.0; Blueberry fruits, 13.0-15.0; Buckleberry fruits, 13.0-15.0; Cowberry fruits, the balance. In 100% of cases the rejuvenescence procedure during the course of treatment improved the state of health, night sleep of patients, promoted visible rejuvenation of the face skin, lowered the arterial hypertension by 10-30 mm Hg, relieved migraine-like headaches, and enhances the male potency (RU 2197256, 2003).

A composition comprising tinctures of different plants with Apilac, Propolis and Nitroglycerin added thereto can be regarded as the most relevant prior art (RU2122424, 1998). Tinctures of the following herbal- and animal-origin materials are used: Arnica, Calendula, Onion, Garlic, Chinese Magnolia-vine, Ginseng, Sicklewort, Plantain offic., Ribwort Plantain, Burdock, Great Mullein, Sage, Shelf Fungus, Dandelion, Cinnamon, Clove, Caraway, Celandine, Curled Dock, Santonica seed, Aloe, Kalanchoe, Oats, sprouted Wheat, Yarrow offic., fly amanita, Echinacea, Black Radish, Bee-moth larvae, Cactus, Hawthorn, Lily-of-the-valley, Yellow Jasmine, Barberry, Ipecacuahna, Digitalis, Valerian, Bulbous Buttercup, Lustwort, Buckthorn Bark, Rauwolfia, colchicum, Spigelia anthelmetica, Malay fishberry, Black Henbane, Marsh Rosemary, Poison ivy, Climbing Sumac, Racemose Baneberry, Cohosh, Iris, Parsley, Dill, Jambolan, White Mistletoe, poison Hemlock, Spurge-flax, Cinchona bark, sea sponge, wild indigo, poppy, hemp, Anacardium Orientale, Lycopodium, Nettle, Gumweed, water Dropwort, drug eyebright, Kidneyroot, White Bryony, Tiger Lily, Juniper, Ignatia, Nux Vomica, Meadow Pasqueflower, Ergot, Uva Ursi.

However, such an wide set of ingredients with different effects may cause allergic reactions and other side effects because of toxic substances that also comprised in the range. Notice should be taken that all the references cited here only for information purposes.

### Disclosure of the Invention

The technical task of the present invention is to develop a composition based on medicinal plants with addition of the main ingredients for correction of the age-related changes: hormone-active and herbal components, as well as to widen the range of agents deprived of allergic reactions and side effects but are capable of correcting age-related changes of the human endocrine system.

The task is accomplished by the invented composition that is able to correct endocrine age-related changes (an anti-aging composition) with an adequate number of component, and, among other things, in micro doses. The formulation includes following components: a composition based on hormone-active and herbal components characterized in that it contains a substance exhibiting hormonal activity (HA) or hormone-like activity (HLA) selected from the group: C21 or C19 steroids, pregnenolone, 17-hydroxypregnenolone, DHEA, progesterone, testosterone, or a hormone-like substance of herbal origin (HS), and also a dry methanolic extract of Nettle root with the following ratio of the components (wt.pts.):

| | |
|---|---|
| HA, HLA or HS | 0.0001-25.0 |
| Nettle root extract | 0.05-50.0 |

The composition contains HS in the form of phytochemical compounds, such as diosgenin and protodioscin in an optimal amount (wt.pts.) 0.0003-0.3.

The anti-aging composition can also contain a dry alcoholic deer antler extract in an amount of 0.00001-10 weight parts. The composition can also further contain a dry extract of ginseng root in an amount of 0.0001-60.0 weight parts.

Very important is the use in our compositions hormonal components in amounts several times less than those used in similar cases.

Our literature searches and experimental investigations have shown that optimal incipients of the composition are precursors of sex hormones, such as phytohormones: from Yam and Tribulus terrestris; C19 and C21 steroids, e.g., dehydroepiandrosterone (DHEA), prednenolone, progesterone or testosterone, a very important component is a Nettle root extract and also a deer antler extract and Ginseng root extract. This statement based on the following data.

Dehydroepiandrosterone is a steroid hormone secreted mainly in the reticular zone of the adrenal cortex.

The use of DHEA in a daily dose of 30-90 mg orally for 4 weeks leads to mood improvement, enhancement of the energy, sexual function and memory improvement in elderly patients (Wolkowits B: Ann. N.-Y. Acad. Sci, 1995; 774:251). This hormone improves the mood, the following effect is observed: DHEA in a dose of 50 mg/day led to an improvement in the physical and mental state in 67% males and 84% females, while the effect of placebo was less than 10% (Morales A.J.: J. Clin. Endocrinol. Metab. 1994; 78:774:251). An angiography demonstrated that in males with 50% and more severe stenosis of coronary arteries level of DHEA is lower than in the control group (Herrington D.M.: Amer. Coll. Card. 1990; 16:862-870). The ability of DHEA to improve the physical state and mental functions in males and females is contributed by the restoration of beta-endomorphine response to specific stimuli.

DHEA reduces hyperglycemia and hyperinsulinemia in diabetic mice (Coleman D.L.: Endocrinology, 1985; 117:2279-2283). A decrease in the insulin resistance upon DHEA administration in 11 females in the post-menopause has also been demonstrated.

The literature data about DHEA with regard to cancerogenesis are contradictory. Treatment with DHEA was associated by the inhibition of tumorogenesis, reduction of the DNA synthesis rate and reduction of the glucose-6-phosphate dehydrogenase activity (Schwartz A.G.: Cancer Res. 1979; 39:1129-1132).

DHEA administered orally in supraphysiological doses (100-300 mg/day) in humans inhibits the synthesis of thromboxane A2 by activated thrombocytes, reduces the amount of the plasma inhibitor of type 1 plasminogen activator and antigen of tissue plasminogen activator, increases the level of the insulin-like growth factor 1 (IGF-1) in blood serum and increases the level of cyclic guanosine monophosphate and the synthesis of nitrogen oxide (directly or by increasing the IGF-1 level). DHEA can play a positive role in immune response modulation. Clinical studies carried out in elderly patients have shown that DHEA in a dose of 50 mg/day increases the IGF-1 level (p < 0.01) and leads to the functional activation of T lymphocytes (increases the content of CD8+, CD56+ cells (natural killers)) and enhances the cytotoxic activity. Serum levels of IL-6 (interleukin6) inversely correlate with the DHEA and DHEA-sulfate (DHEA-S) level in blood serum (p < 0.001). Besides, DHEA, DHEA-S, androstenedione inhibit the production of IL-6 by peripheral mononuclears in a dose-dependent manner (p < 0.01).

The DHEA half-life period is 15-30 minutes, and the metabolic clearance rate (MCR) is 2000 L/day, while in DHEA-S the half-life period is significantly longer and is about 7-10 hours, and the MCR is 5-20 L/day.

DHEA easily absorbed after PO use. The distribution volumes for DHEA and DHEA-S are 17-38.5 L and 8.5-9.3 L respectively. DHEA and DHEA-S are converted into some active metabolites, including androstenedione, testosterone, estrone, estradiol, estriol. The DHEA half-life elimination period is 15-38 minutes, while the DHEA-S half-life period is 7-22 hours. Renal excretion is 51-73% of the elimination of DHEA-S and its metabolites.

Deer antler extract has been intensively studied and widely used in Russia since 1930s, mainly as an alcoholic extract. In recent years the interest to this long time known medicinal agent has grown owing to the results of unique research programs carried out in New Zealand. Deer antler extract is a natural source of IGF-1 located in a biologically active matrix. The present-day preparation, pantocrine, is obtained from Siberian Deer, Siberian Stag or red Deer Antler velvet. Pantocrine contains 18 of the known 22 natural amino acids, among which glycine, alanine, proline, leucine, tryptophan, cystein, lysine, hystidine, threonine are in a predominant amount. A lipoid fraction is predominant in pantocrine and contains lipids, phosphatides, sulfatides, cerebrosides, sterols, and their derivatives. In the lipoid fraction steroids are presented mainly by cholesterol which is the natural substance and precursor for biologically substances like bile acids, vitamins and hormones: estrogen, estrone, estriol) and androgens (testosterone, androsterone). Pantocrine contains various pharmacologically active substances, such as male and female sex hormones, a significant amount of electrolytes, active protein and nitrogenous fractions. Besides, the preparation contains phospholipids and trace elements, it enhances the working capacity. Pantocrine can be administrated orally and parenterally (subcutaneously and intramuscularly).

Pantocrine is used as a tonic for exhaustion of different genesis, arterial hypotonia, and a natural source of IGF-1 and of growth hormones, enhances the energy potential, muscular power and endurance, improves physical and mental (overall) health, stimulates immunity, alleviates joint pain and enhances motor ability.

A large number of substances has been isolated from Nettle root with different polarity and from different chemical classes, including fatty acids, terpenes, phenyl propanes, lignans, coumarins, triterpenes, ceramides, sterols, and lectins. Among them: oxalic acid, linolenic acid, 14-octacosanol, 13-hydroxy-9-cis, 11-trans-octadecanoic acid, alpha-dimorphecolic acid (9-hydroxy-10-trans, 12-cis-octadecadinoic acid), scopoletin, p-hydroxybenzaldehyde, homovanilinebutyric alcohol, beta-sitosterol, stigmasterol, 24-R-ethyl-5-alpha-cholestane-3-beta,6-alpha-diol, campesterol, daucosterol (and allied glycosides), secoisolaricerosinol-9-O-beta-D-glycoside, neoolivile, oleanoic acid, ursolic acid, Urtica Dioica agglutinin and polysaccharides RP1-RP5.

The age-related reduction of the ACTH (adrenocorticotropic hormone) level can be compensated with use of panaxosides - substances contained in the root of Panax Ginseng. Ginseng use enhances the activity of brain cells which produce the adrernocorticotropic hormone (ACTH). ACTH stimulates the production of adrenal hormones in stress situations. This activity of Ginseng is conditioned by saponins (ginsenosides) and by the element germanium. A mixture of saponins or isolated ginsenosides extracted from the root of Panax ginseng (panaxosides) upon administration to rats increases the level of ACTH and CRF in 30, 60, 90 minutes. The dynamics of increase of plasma ACTH is almost parallel to that of plasma corticosteroids. Isolated ginsenosides, protopanaxadiol or propanaxatriol glycosides also increased the levels of corticosteroids in plasma. The ginseng-induced increase of corticosteroids in plasma is inhibited by preliminary administration of dexametasone. It has been shown that the main action of ginseng saponins on the hypothalamus and/or hypophysis is by stimulation of ACTH secretion which leads to raise in the synthesis of coticosteroids in the adrenal cortex of rats (J. Clin. Endocrinol. Metasbol., 2001; 86(11)).

There are also herbal precursors for sex hormones- steroid saponins that have a similar chemical structure with natural precursors of steroid hormones (phytosterols, tetracycliclic lipophilic triterpene derivatives), for example, saponins diosgenin and protodioscin, produced from plants of the Dioscoreaceae family, including Dioscorea villosa, D. opposita, D. hypoglauca, D. composita, D. deltoida, D. parazeri, D. mastrostachya, D. floribunda, D. barbasco, D. mastrostachya; Tribulus terrestris.

Dioscorea preparations have long been used in medicine. Thus, in the British Pharmacopoeia spasmolytic, anti-inflammatory, anti-rheumatic and choleretic effects of dioscorea are described, and the following indications for use are given: treatment of intestinal colic, diverticulitis, rheumatoid arthritis, cholecystitis, intermittent claudication, dysmenorrhea etc. Besides, there are data available regarding the beneficial effect of diosgenin (dioscorea saponin) on the level of cholesterol. However, we suggest to use dioscorea preparations in a new quality: for the normalization and rejuvenescence of the hormonal system of an organism.

Hence, studied main components contribute to invention of anti-aging composition, are necessary and sufficient to obtain the required technical task as compared with the numerous known polycomponent formulations cited above (see the abovementioned patent referrals).

***The inventive composition is prepared in the following manner**:* preliminarily prepared batches of dry components are dissolved in ethanol, thoroughly mixed, then filtered to remove foreign particles with consequent evaporation of the solvent. The resulting powder is dried and used for preparation of the pharmaceutical form suitable for a particular patient; the preparation of such form being one more subject of the present invention. The examples of the inventive composition are presented below (see the Examples) as particular forms for use.

Still another subject (variant) of the present invention is a variant of the composition for correcting age-related changes of human endocrine system. We consider that the most relevant prior art is an invention having a similar effect, based on herbal preparations. For instance, French patent No. 2671488, 1992: a composition containing a Ginseng extract and magnesium salts.

Our composition contrary to the known compositions contains a dry Ginseng extract and addition of dry Nettle root extract with the following ratio of the components (weight parts);

| | |
|---|---|
| Ginseng extract | 0.0001-60.0 |
| Nettle root extract | 0.005-50.0 |

The use of this composition has shown its appreciably higher efficiency (18-55% over placebo) in terms of improving endocrine characteristics, both visible (small wrinkles disappeared, the skin acquired elasticity) and internal: the level of hormones in blood approaches near to that characteristic of young age.

The method for production of a pharmaceutical form for use of the claimed compositions includes the following steps: mixing an active component and pharmaceutically acceptable carrier, when the active component is a dry mixture (composition) and at least 1.5% of the total weight of the final form. For producing liquid pharmaceutical forms, dry extracts are preliminarily dissolved in ethyl alcohol and then mixed with other fillers and adjuvants.

The form contains, as the carrier, hard, soft or liquid substances and comprises a tablet, dragee, powder, granule, sachet, chewable tablet or troche, capsule, pill, solution, gel, emulsion, potion, syrup, liniment, ointment, cream, paste, suppository or implant.. Solid carrier may contain suitable filers, binders, and humidifiers, and, when required, dispersers, moisturizers and other components. Solid forms may be coated with by using suitable known procedures. Soft forms are prepared by using known oil bases (ointments, pastes) or plastic masses on a suitable carrier (implants, plasters).

Liquid forms may contain suitable additives: suspending agents, salts, emulsifiers, humectants, non-aqueous solvents (including edible oils), preservatives in accordance with the XI edition of State Pharmacopoeia, as well as flavors and/or food colorants.

Correspondingly, the resulting pharmaceutical form may be administered orally, sublingually, intranasally, subconjunctivally, rectally, vaginally, parenterally.

The resulting pharmaceutical forms are used individually, in an amount efficient for a particular patient, in accordance with the patient's age, weight, and other personal traits.

### Herein below we present particular examples of the claimed composition as pharmaceutical forms prepared according to our method.

**EXAMPLE 1.** A liquid form for injections. The composition comprises the following formulation:

| | |
|---|---|
| DHEA | 5.0 mg |
| Nettle root extract | 0.25 g |
| Water for injections | to 100 ml |

The preparation is dispensed in 1 ml single-use syringes, sterilized and packed. Each syringe contains one dose for injection, which is a part of a daily dose.
**EXAMPLE 2**. A liquid form as an aqueous solution for oral administration:

| | |
|---|---|
| Nettle root extract | 0.5-5.0 g |
| Diosgenin or protodioscin | 0.03-0.3 g |
| Distilled water | to 100 ml. |

Appropriate amount of each ingredient are taken, dissolved in 1-3 ml of ethanol, The resulting solution is diluted to 100 ml with distilled water.
**EXAMPLE 3.** An oily form for external use.

| | |
|---|---|
| DHEA | 100-250 mg |
| Nettle root extract | 300-500 mg |
| Dry Deer Antler velvet extract | 0.001-10 mg |
| Dry Ginseng root extract | 0.006-6.0 mg |
| Purified sterilized olive oil | to 100 ml. |

Ingredients are preliminarily dissolved in ethanol and than thoroughly stirred in oil. The form is used externally, intranasally, vaginally, etc.
**EXAMPLE 4**. A soft oil-based form for external use:
DHEA, Nettle root extract, a dry Deer Antler velvet extract, a dry Ginseng root extract taken in amounts as in Example 3.
The resultant batches, starting with the smallest, are mixed with addition of oil base consisting of vaseline, lanoline and spermacet, taken in equal parts.
**EXAMPLE 5**. A soft pharmaceutical form which is a paste for topical applications:

| | |
|---|---|
| Nettle root extract | 270 mg |
| Diosgenin or protodioscin | 0.3 mg |
| Dry Deer Antler velvet extract | 30 mg |
| Vaseline | 30 mg |
| Starch | 20 g. |

**EXAMPLE 6.** A solid pharmaceutical form which is a tablet for oral use.

| | |
|---|---|
| DHEA | 25 mg |
| Dry Deer Antler extract | 10 mg |
| Dry Ginseng root extract | 60 mg. |

This rating is cited for one 0.6 g factory-made tablet, produced by following a standard procedure with the use of lactose, starch, gelatin, sodium chloride and adding a required amount of water and other adjuvants.
**EXAMPLE 7.** A solid pharmaceutical form which is a powder placed into a gelatin capsule:

| | |
|---|---|
| Diosgenin or protodioscin | 0.03 mg |
| Nettle root extract | 5.0 mg |
| Dry Deer Antler velvet extract | 5.0 mg |
| Dry Ginseng root extract | 15.0 mg. |

Dry components are carefully placed into one capsule. The abovementioned calculations are for one use. For each patient the number and doses of the components are selected individually.
The preparation of a pharmaceutical form for the variant of the composition (Nettle root extract + Ginseng root extract) has the same principle as that for the main composition.
*Now we present examples of pharmaceutical form for the composition.*
**EXAMPLE 8.** A solid pharmaceutical form which is a mixture of a dry Nettle root extract and a dry Ginseng root extract, placed into a gelatin capsule.

| | |
|---|---|
| Nettle root extract | 0.3 g |
| Ginseng root extract | 0.006 g. |

**Example 9**. A liquid pharmaceutical form for internal use.

| | |
|---|---|
| Nettle root extract | 0.005 g |
| Ginseng root extract | 0.2 g |
| Normalized drinking water | to 10.0 g |

The biological activity of the composition has been checked both under experimental and clinical conditions, as is supported by the following Examples.
**EXAMPLE 10.** An investigation of the hormonal profile of the blood of laboratory rats and monitoring of their behavior showed the following. 3 groups of experimental animals (10 rats in each group) were used in the study. A control group was kept in conventional vivarium conditions. A comparison group additionally received substances used as fillers for preparing pharmaceutical forms: starch, vegetable oils, lactates, stearates etc. A study group was administered subcutaneous injections of the composition described in Example 1 once a day during one month. The results of the investigations have shown not only a quantitative shift of the hormonal levels toward those characteristic of young animals by 70-85% (p < 0.05), but also behavioral reactions of the animals of both sexes.
Clinical investigations on volunteer patients have shown as follows. 22 individuals participated in the experiment: 11 males and 11 females aged from 36 to 65. The results have shown that the use of the invented composition in 97.5% of cases (p ≤ 0.05) leads to the rejuvenescence of the hormonal profile to the level of humans aged from1 8 to 25.
*Examples of testing the claimed compositions on particular patients support this fact.*
**EXAMPLE** 11. Male patient A.B., aged 36, looks older for his age. Complaints: loss of sexual potency. A comparative analysis of the hormonal profile before and after the course of treatment with pharmaceutical forms cited in Example 2 for 30 days showed significant changes. After the course of treatment the patient's mood improved, the skin turgor increased. The patient looked younger and no longer complained of sexual impotence. Besides, while the initial level of testosterone in blood was 16.4 nmol/L or only 14% of an average for the age under consideration, after the course of treatment the level of this hormone grew to 30.1 nmol/L or about 160% of an average for his age group. The level of another important hormone, adrenocorticotropic hormone (ACTH), in the patient's blood after the course of treatment approached to that for 18-20 yrs, i.e., 30.2 pg/L. (with the initial level in this 36-year old patent the ACTH level was 10.3 pg/lit. (60-65 yrs).
**EXAMPLE 12**. Male patient B. S., aged 62, healthy but liked to look younger and enhance libido. The patient received a course of treatment with composition (taken orally) according to Example 7. The patient received 1 capsule once in the morning. On completion of the course the patient felt more energetic, his insomnia disappeared and libido enhanced and all these accompanied by improvement of the elasticity of the muscles and skin. An analysis of hormonal parameters has supported the fact of the anti-aging (rejuvenizing) effect of the composition. The hypothalamus hormones have reached a level of 40-45 years; some hypophyseal hormones have reached a level corresponding to 20-25 yrs; some sex hormones have even reached a level corresponding to 18-20 yrs.
**EXAMPLE 13**. Male patient S.P., 37 y.o. with age-related changes of the level of male sex hormones associated with reduced libido and visible initial stages of skin senility; the patient complains of fatigue at the end of the working day and on exercises. During 1 month the patient received the preparation described in Example 8 twice a day. Initially, a low level of dihydrotestosterone, free testosterone, and a high level of steroid-binding globulin (SBG) were observed. After the course of treatment with our composition (based on a Nettle root extract) the decrease in SBG level was observed with a concomitant increase of the level of total testosterone, free testosterone and dihydrotestosterone - most active male sex hormones. In particular, the level of free testosterone has increased from 5.3 pg/ml to 13.0 pg/ml, the latter value corresponding to the level of this hormone for males aged 20-25. The complaints mentioned above disappeared, wrinkles on the face smoothed out.
**EXAMPLE 14**. Male patient D.A., aged 36, complained of easy fatigability and weakness. According to the biochemical hormonal analysis data, the patient has low levels of DHEA, adrenocorticotropic hormone, and some releasing hormones. After 1 month of treatment with our pharmaceutical form (Example 9) twice a day, essential changes took place in the patient's endocrine system. These changes were accompanied by an increase of the DHEA level from 16.0 nmol/L. to 26.6 nmol/L. (18-20 yrs) and of the ACTH level from 0.8 pg/L to 30.2 pg/L (corresponds to 18-20 yrs); other hormonal indexes are also normalized. An improvement of the overall health was noted, tolerance to physical loads increased.

Hence, the new compositions, as well as an individualized method for preparation of pharmaceutical forms based on said compositions are very effective in correction of human endocrine system, rejuvenation of patients that represented by both external traits and the hormonal profile. Use of the invention broadens the range of medicinal agents of natural origin, which allow to correct age-related hormonal changes in human organism.

## Claims

1. A composition for correction of age-related changes of the human endocrine system including hormone-active and herbal components, **characterized in that** it contains a substance with hormonal activity (HA) or with hormone-like activity (HLA) selected from the group: C21 or C19 steroids, pregnenolone, 17-hydroxypregnenolone, DHEA, progesterone, testosterone, or a hormone-like substance of herbal origin (HS), and also a dry methanolic extract of Nettle root with the following ratio of the components (weight parts):
| | |
|---|---|
| HA, HLA or HS | 0.0001-25.0 |
| a Nettle root extract | 0.05-50.0. |

2. The composition of claim 1, **characterized in that** it contains HS in the form of phytochemical compounds such as diosgenin or protodioscin.

3. The composition of claim 1, **characterized in that** it further contains a dry Deer Antler velvet extract in an amount of 0.00001-10.0.

4. The composition of claim 2, **characterized in that** it further contains a dry Deer Antler velvet extract in an amount of 0.00001-10.0

5. The composition of claim 2, **characterized in that** it further contains a dry Ginseng root extract in an amount of 0.00001-60.0.

6. The composition of claim 3, **characterized in that** it further contains a dry ginseng root extract in an amount of 0.00001-60.0.

7. A composition for correcting age-related changes (a variant), comprising a hormone-like substance of herbal origin, **characterized in that** it contains a dry Ginseng root extract and further contains a dry Nettle root extract with the following ratio of the components (weight parts):
| | |
|---|---|
| Ginseng extract | 0.0001-60.0 |
| Nettle root extract | 0.005-50.0. |

8. A method for production of a pharmaceutical form based on the composition **characterized in** claims 1-6 or 7, the method includes the mixture of an active component and pharmaceutically acceptable carrier with suitable adjuvants differs by use composition **characterized in** claims 1-6 or 7 as an active component in an effective amount.

9. The method for producing a pharmaceutical form of claim 8, **characterized by** the introduction of an active component into the formulation in a concentration of no less than 1.5%.

10. The method of claim 8, **characterized in that** solid, soft or liquid substances are used as the carrier.

11. The method of claim 8, **characterized in that** the form is produced with the use of solid carriers, wherein the end form is a tablet, a dragee, a granule, a sachet, or a powder placed into a capsule.

12. The method of claim 8, **characterized in that** the form is produced with the use of liquid carriers, wherein the end product is a solution, a gel, an emulsion, a suspension, a potion, a syrup or a liniment.

13. The method of claim 8, **characterized in that** the form is produced with the use of soft carriers, wherein the end product is an ointment, a crème, a paste, a suppository, an implant, or a chewing tablet or a troche.

14. The method of claim 8, **characterized in that** the produced form is used in a suitable manner, selected from the group consisting of oral administration, sublingual administration, intranasal administration, rectal administration, vaginal administration, parenteral administration, subconjunctival administration, or a chewable form.
